# EUROPÄISCHE PATENTSCHRIFT

(11) **EP 3 525 686 B1**
(45) Veröffentlichungstag und Bekanntmachung des Hinweises auf die Patenterteilung: **30.09.2020**
(21) Anmeldenummer: 17800361.2
(22) Anmeldetag: 17.10.2017
(51) Int. Cl.: A61B 17/068

(54) **VORRICHTUNG ZUM VERBINDEN VON KÖRPERGEWEBEN**
DEVICE FOR CONNECTING BODY TISSUES
DISPOSITIF POUR RELIER DES TISSUS CORPORELS

(30) Priorität: 17.10.2016 AT 509322016
(43) Veröffentlichungstag der Anmeldung: 21.08.2019
(73) Patentinhaber: Klaffenböck, Johann, 5350 Strobl (AT); Klaffenböck, Lukas, 5350 Strobl (AT); Mair, Julian, 81925 München (DE)
(72) Erfinder: Klaffenböck, Johann, 5350 Strobl (AT); Klaffenböck, Lukas, 5350 Strobl (AT); Mair, Julian, 81925 München (DE)
(74) Vertreter: Babeluk, Michael
(86) Internationale Anmeldenummer: PCT/AT2017/060264
(87) Internationale Veröffentlichungsnummer: WO 2018/071932

(56) Entgegenhaltungen:
- EP-A1- 0 040 157
- EP-A2- 0 085 931
- WO-A1-2009/135005
- DE-T2- 3 689 806
- US-A- 5 240 164
- US-A- 5 564 615

## Beschreibung

Die vorliegende Erfindung betrifft eine Vorrichtung zum Verbinden von Körpergeweben gemäß dem Oberbegriff von Patentanspruch 1.

Es ist in der Medizin bekannt, Wunden durch Gewebeklammern zu verschließen, die im Bereich der Wundränder in die Haut eingeführt werden und danach verformt werden, um die Wundränder gegeneinander zu fixieren. Zur Durchführung dieses Vorgangs sind Vorrichtungen entwickelt worden, die im allgemeinen Stapler genannt werden. Solche Vorrichtungen sind beispielsweise in der US 5 170 926 A, der EP 1 695 668 A oder der EP0 354 724 B beschrieben. Typischerweise werden dabei mehrere Gewebeklammern in einem Magazin gestapelt, ähnlich wie dies bei Büroklammern der Fall ist. Vor der Benutzung wird die jeweils vorderste Gewebeklammer in eine Stellung gedreht, in der sie vorgeschoben und in die Haut eingebracht werden kann.

Die vorliegende Erfindung betrifft Vorrichtungen, die zur Verbindung von Abschnitten von Körpergeweben in der oben beschriebenen Art geeignet sind, jedoch mit dem Unterschied, dass die Vorrichtung hinreichend klein und kompakt ausgebildet ist, um in künstliche oder natürliche Körperöffnungen eingeführt zu werden, um Körpergewebe im Inneren des Körpers zu verbinden. Es geht also um die Benutzung in endoskopischen oder laparoskopischen Eingriffen. Die besondere Anforderung besteht somit darin, die Vorrichtung mit einem möglichst geringen Querschnitt auszubilden, die erforderlichen Betätigungskräfte sicher einzuleiten und eine hohe Zuverlässigkeit zu gewährleisten, da eine direkte visuelle und taktile Kontrolle bei dieser Form der Anwendung nicht möglich ist.

Es sind verschiedene Vorrichtungen zum Verbinden von Körpergeweben im Inneren des Körpers bekannt geworden, nämlich aus der DE 36 89 806 T2, der WO 2009/135005 A1, der US 5,564,615 A, der EP 85 931 A2 und der EP 40 157 A1. Diese Vorrichtungen sind jedoch aufwändig in der Anwendung und vielfach nur für besondere Anwendungsfälle geeignet.

Aufgabe der vorliegenden Erfindung ist es, eine einfache, zuverlässige und problemlos anwendbare Vorrichtung zu schaffen, mit der es möglich ist, Körpergewebe im Inneren des Körpers zu verbinden.

Erfindungsgemäß werden diese Aufgaben durch eine Vorrichtung gemäß Patentanspruch 1 gelöst.

Es ist ein wesentlicher Aspekt der vorliegenden Erfindung, dass die einzelnen Gewebeklammern aktiv durch Rotorelemente aus der Ebene, in der sie im Magazin gestapelt sind (Vorratsstellung) in die Ebene gedreht werden, in der sie vorgeschoben werden, um in das Körpergewebe einzudringen (Arbeitsstellung).

Die Rotation erfolgt dabei um eine Achse, die senkrecht zur Längsachse der Vorrichtung und zur Vorschubrichtung der Gewebeklammern ist.

Ein weiterer wichtiger Aspekt der vorliegenden Erfindung besteht darin, dass ein Schieber sowohl die Aufgabe übernimmt, eine Gewebeklammer vorzuschieben, als auch die Aufgabe, diese nach dem Eindringen in das Körpergewebe zu verformen, um eine sichere Verbindung herzustellen. Auf diese Weise kann die Anzahl der benötigten Bauteile gering gehalten werden und die Vorrichtung besonders kompakt gestaltet werden.

Es ist günstig, wenn jeweils eine Rotorfeder an einem Rotorelement angreift, um dessen Bewegung in zwei Rotationsstellungen einrastbar auszuführen, und dass vorzugsweise die Rotorelemente mit einer Welle verbunden sind. Auf diese Weise ist es möglich, den Antrieb der Rotorelemente einfach und mit ausreichenden Toleranzen gestalten zu können, da die exakte Winkellage der Rotorelemente in den kritischen Stellungen, nämlich der Aufnahme und der Abgabe der Gewebeklammern durch das Einrasten gewährleistet ist.

Eine besonders elegante Möglichkeit, den Vorschub der Gewebeklammern im Magazin und die notwendige Auflagekraft an den Rotorelementen sicherzustellen besteht darin, dass die Gewebeklammern in Vorratsstellung durch zumindest eine Vorratsfeder über einen Vorratsschlitten gegen das Rotorelement vorgespannt wird, wobei die Vorratsfeder vorzugsweise an der Welle der Rotorelemente befestigt ist. Die Vorratsfeder ist dabei eine Blattfeder, die in die aufgewickelte Stellung vorgespannt ist. Sie hat dabei stets das Bestreben, den linearen Abschnitt parallel zum Magazin zu verkürzen und sich um die Welle aufzuwickeln. Der besondere Vorteil dieser Lösung besteht darin, dass die Kraft, mit der der Vorratsschlitten beaufschlagt wird, unabhängig von der Stellung des Vorratsschlitten ist, also von der ersten bis zur letzten Gewebeklammer im Wesentlichen gleich bleibt.

Ein besonders hoher Grad an Kompaktheit kann dadurch erreicht werden, dass die Rotorelemente durch zumindest ein Pleuel angetrieben werden, das an ihnen angelenkt ist, und welches vorzugsweise über einen Verbindungsschieber mit dem Schieber in Verbindung steht. Auf diese Weise ist es möglich, mit einem einzigen Antriebselement sowohl den Schieber als auch die Rotorelemente anzutreiben, wodurch auch zusätzlicher Aufwand für eine allfällige Synchronisierung unterschiedliche Antriebselemente entfällt.

Insbesondere ist es in diesem Zusammenhang günstig, wenn die Rotorelemente durch je ein Pleuel angetrieben werden, und vorzugsweise je über einen Verbindungsschieber mit dem Schieber in Verbindung stehen. Auf diese Weise wird eine symmetrische Krafteinleitung gewährleistet.

Eine optimale Abfolge der einzelnen Bewegungen wird insbesondere dadurch erreicht, dass der bzw. die Verbindungsschieber fest mit dem Schieber verbunden ist, und dass an jedem Verbindungsschieber ein Langloch angeordnet ist, in das je ein Zapfen des Pleuels eingreift.

Durch das Langloch wird erreicht, dass in einer ersten Phase der Vorwärtsbewegung nur der Schieber vorgeschoben wird, während der Zapfen des Pleuels im Langloch des Verbindungsschiebers nach hinten gleitet, so dass das Pleuel und damit die Rotorelemente nicht bewegt werden. Diese Phase ist dann beendet, wenn der Zapfen am hinteren Ende des Langlochs angekommen ist. In einer zweiten Phase der Vorwärtsbewegung nimmt der Verbindungsschieber nunmehr das Pleuel mit und bewirkt auf diese Weise eine Verdrehung der Rotorelemente.

Bei der Rückwärtsbewegung ist in analoger Weise zunächst nur eine Bewegung des Schiebers zu beobachten, während der Zapfen des Verbindungsschieber im Langloch nach vor gleitet, so dass auch hier keine Bewegung von Pleuel und Rotorelementen stattfindet. Erst dann, wenn der Zapfen am vorderen Ende des Langlochs anliegt, wird das Pleuel mitgenommen und es werden die Rotorelemente zurückgedreht.

Eine besonders bevorzugte Ausführungsvariante der vorliegenden Erfindung sieht vor, dass ein Schieber auf der, den Eingriffsabschnitten abgewandten, Seite der Hauptabschnitte der Gewebeklammern in Richtung der Längsachse beweglich angeordnet ist, der an seiner vorderen Stirnseite zwei seitliche Vorschubbereiche aufweist, zwischen denen eine, im Wesentlichen rechteckige, Ausnehmung vorgesehen ist, und dass ein Rückhalteelement vorgesehen ist, das in die Ausnehmung des Schiebers eingreift, um beim Vorschub des Schiebers den linearen Hauptabschnitt einer dazwischenliegenden Gewebeklammer zu verformen.

Es ist ein wichtiger Aspekt der vorliegenden Erfindung, dass der Schieber sowohl den Vorschub der Gewebeklammern als auch deren Verformung in einer einzigen Bewegung bewerkstelligt. Günstig ist dabei, dass die Kraft auf die jeweilige Gewebeklammer beim Vorschub direkt im Bereich der Eingriffselemente ausgeübt wird, so dass diese gegen den Widerstand des Körpergewebes vorgeschoben werden können, ohne zunächst eine Verformung der Gewebeklammer zu verursachen. Erst dann, wenn die Gewebeklammer ausreichend tief in das Körpergewebe eingedrungen ist, kommt der mittlere Abschnitt des Hauptabschnitts der Gewebeklammer in Anlage an das Rückhalteelement, so dass die Gewebeklammer um die Kanten des Rückhalteelements umgebogen wird.

Nachdem die Gewebeklammer im Körpergewebe ordnungsgemäß gesetzt worden ist, muss eine sichere Ablösung von der erfindungsgemäßen Vorrichtung gewährleistet werden. Gemäß einer bevorzugten Ausführungsvariante der vorliegenden Erfindung erfolgt dies dadurch, dass eine Auswurffeder so angeordnet ist, dass diese eine verformte Gewebeklammer vom Rückhalteelement abstreift. Ein besonderer Vorteil dieser Lösung besteht darin, dass das Abstreifen der Gewebeklammer vollautomatisch geschieht, ohne einen besonderen Steuerungsaufwand zu verursachen.

Das Abstreifen wird auf besonders einfache Weise dadurch bewerkstelligt, dass die Auswurffeder im kräftefreiem Zustand in den Bewegungsbereich des Schiebers ragt. Durch das Vorschieben des Schiebers wird die Auswurffeder vorgespannt und durch das Zurückziehen des Schiebers wird die Feder freigegeben, um die Gewebeklammer abzustreifen.

Eine besonders sichere Führung der Gewebeklammer kann dadurch erreicht werden, dass am Schieber eine Nase vorgesehen ist, der die vorzuschiebende Gewebeklammer gegen die Vorspannung der Auswurffeder abstützt.

Eine optimale Konfiguration der Gewebeklammer in appliziertem Zustand wird dadurch erreicht, dass die Ausnehmung im Wesentlichen rechteckig ist.

Eine besonders kompakte und Platz sparende Ausbildung der erfindungsgemäßen Vorrichtung kann dadurch erreicht werden, dass der Schieber plattenförmig ausgebildet ist und der Bewegungsbereich des Schiebers in einer Ebene liegt, die im Bereich der Spitzen der Eingriffsabschnitte der Gewebeklammern in Vorratsstellung liegt, und besonders vorzugsweise im Wesentlichen parallel und mit konstantem Abstand zu den Hauptabschnitten der Gewebeklammern in Vorratsstellung liegt. Auf diese Weise ist es möglich, dass der Schieber und andere Bauteile, die dem Antrieb des Schiebers dienen, den Raum nutzen, der durch die im Magazin im Vorratsstellung vorliegenden Gewebeklammern aufgespannt wird.

Ein besonders effizienter Betrieb der Vorrichtung wird dadurch erreicht, dass der Schieber eine vordere Stellung und eine hintere Stellung besitzt, wobei in der vorderen Stellung zwischen dem Schieber und dem Rückhalteelement eine verformte Gewebeklammer eingeklemmt ist, und in der hinteren Stellung eine aus der Vorratsstellung in eine senkrecht daran liegende Arbeitsstellung gedrehte Gewebeklammer aufnehmbar ist.

Ein wichtiger Aspekt der vorliegenden Erfindung ist insbesondere dadurch gegeben, dass ein Hydraulikzylinder und/oder eine Kolbenstange zumindest teilweise im Inneren des Raums angeordnet sind, der zwischen den Eingriffsabschnitten der Gewebeklammern in der Vorratsstellung liegt, und vorzugsweise, dass die Kolbenstange mit dem Schieber fest verbunden ist.

Wesentlich dabei ist zunächst der hydraulische Antrieb über einen Hydraulikzylinder, der unabhängig von der Entfernung zu einem Bedienungselement die Aufbringung der erforderlichen Kräfte ermöglicht. Da die Gewebeklammern in der Vorratsstellung den Hydraulikzylinder bzw. die Kolbenstange teilweise umgeben wird eine besondere Platzersparnis erreicht.

Insbesondere kann die Vorrichtung dadurch besonders kompakt ausgestaltet werden, dass der Hydraulikzylinder doppelwirkend ausgeführt ist.

Eine besonders begünstigte Ausführungsvariante der Erfindung sieht mindestens einen Kanal zur Aufnahme eines Halteinstruments vor. Solche an sich bekannten Halteinstrumente können vorteilhaft dazu benutzt werden, Gewebe festzuhalten oder in ihrer Lage zu manipulieren, um die Gewebeklammern optimal einsetzen zu können.

Vorzugsweise sind zwei Kanäle vorgesehen, die vorzugsweise nach vorne geringfügig auseinanderlaufen, wobei besonders vorzugsweise der Winkel zwischen den Kanälen steuerbar ist. Dadurch kann entfernteres Gewebe passend herangezogen werden.

Insbesondere kann die Manipulation des Körpergewebes dadurch besonders flexibel gestaltet werden, wenn die Halteinstrumente unabhängig voneinander steuerbar sind.

Eine besonders kompakte Konstruktion der erfindungsgemäßen Vorrichtung kann insbesondere dadurch erreicht werden, dass der Kanal vorzugsweise benachbart zum Hydraulikzylinder angeordnet ist, und/oder dass der Kanal zwischen den Seitenteilen angeordnet ist.

Besonders günstig ist es, wenn der Kanal im proximalen Bereich des Kopfteils angeordnet ist. Dadurch bleibt unterhalb der Gewebeklammern in ihrer Vorratsstellung Platz, so dass sich die Halteinstrumente aus den divergenten Kanälen seitlich auch außerhalb des Querschnitts der Vorrichtung vorgeschoben werden können, um auch laterale Bereiche des Körpergewebes erfassen und in den Bereich der Gewebeklammern ziehen zu können.

In der Folge werden Ausführungsbeispiele der vorliegenden Erfindung anhand der beigefügten Figuren näher erläutert. Es zeigen:
- Fig. 1: eine erste Ausführungsvariante einer erfindungsgemäßen Vorrichtung in einer Ansicht von unten;
- Fig. 2: die Vorrichtung von Fig. 1 in einer Seitenansicht;
- Fig. 3: die Vorrichtung von Fig. 1 und Fig. 2 in einer in einer Ansicht von oben;
- Fig. 4: die Vorrichtung von Fig. 1 bis Fig. 3 in einer in einer Vorderansicht;
- Fig. 5: die Vorrichtung von Fig. 1 bis Fig. 4 in einer Explosionsdarstellung;
- Fig. 6: bis Fig. 9 Längsschnitte in verschiedenen Stadien der Bewegung zur Erklärung der Funktion der Vorrichtung;
- Fig. 10: und Fig. 11 unterschiedliche Schrägansichten einer ersten Ausführungsvariante des Rotorelements;
- Fig. 12: und Fig. 13 unterschiedliche Schrägansichten einer zweiten Ausführungsvariante des Rotorelements;
- Fig. 14: eine weitere Ausführungsvariante einer erfindungsgemäßen Vorrichtung in einem Längsschnitt;
- Fig. 15: und Fig. 16 zwei alternative Einsatzmöglichkeiten der Vorrichtung jeweils in einer Schrägansicht;
- Fig. 17: und Fig. 18 jeweils einen Längsschnitt einer weiteren Ausführungsvariante der Erfindung in zwei unterschiedlichen Stellungen; und

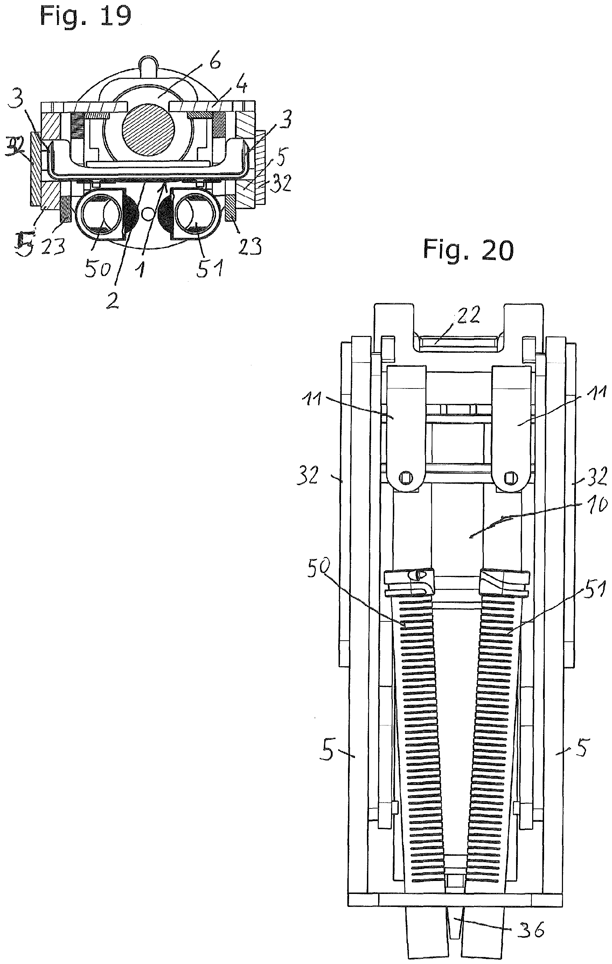

- Fig. 19: einen Querschnitt durch die Ausführungsvariante von Fig. 17 und Fig. 18,
- Fig. 20: eine Ansicht der Ausführungsvariante von Fig. 17 bis Fig. 19 von unten.

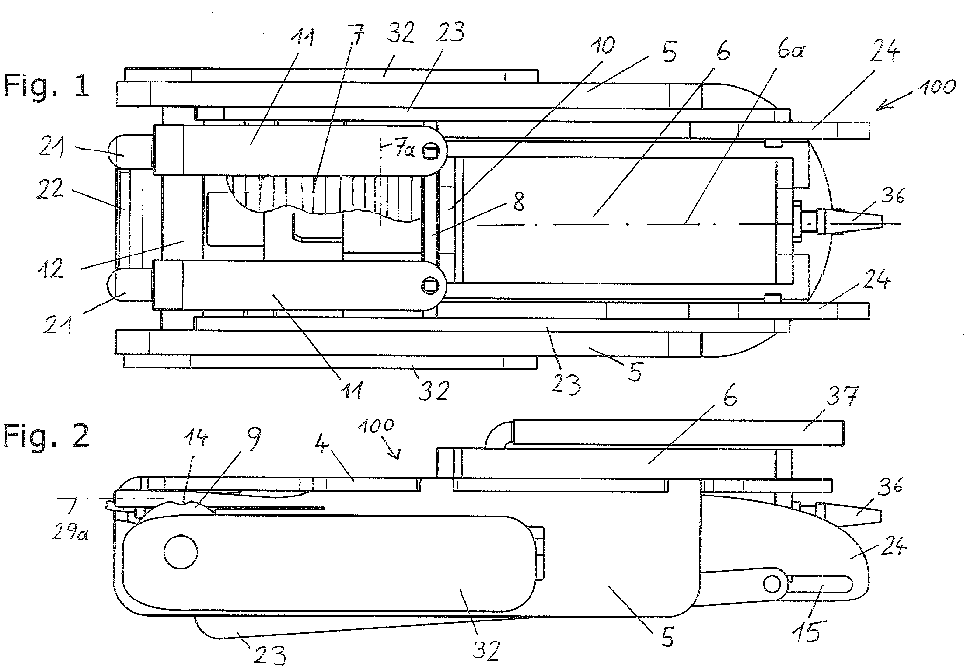

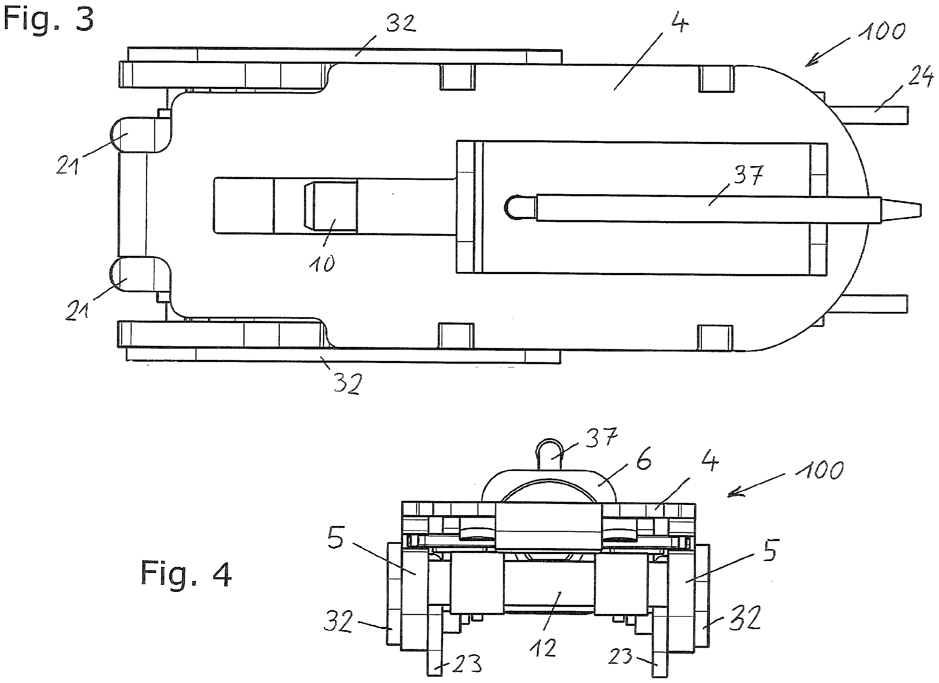

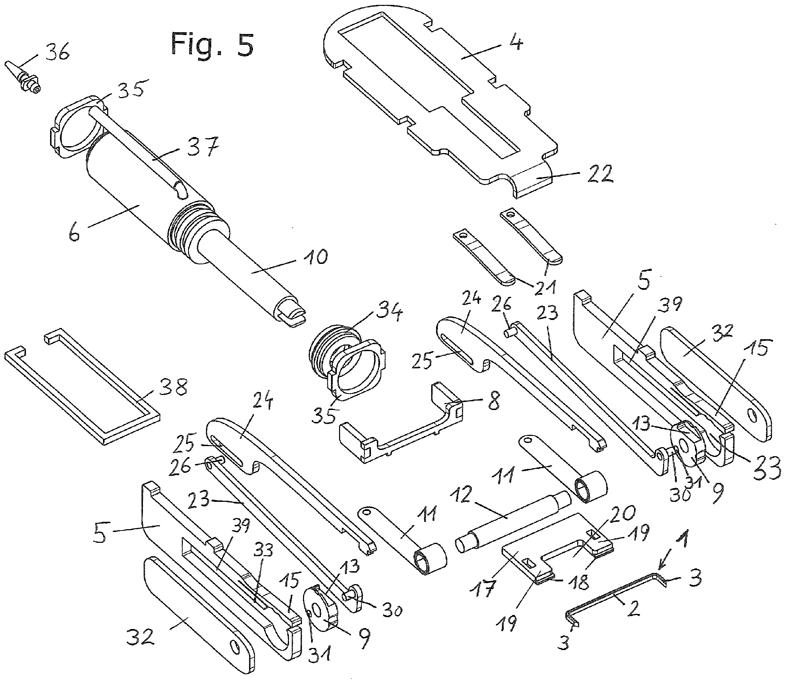

Die Fig. 1 bis Fig. 5 zeigen den Kopfteil 100 einer erfindungsgemäßen Vorrichtung, die eine Gewebeklammer 1 mit einem linearen Hauptabschnitt 2 und zwei davon senkrecht abstehenden, parallelen Eingriffsabschnitten 3 (ersichtlich in Fig. 5) ausstoßen und verformen kann.

Die Vorrichtung weist ein Trägerelement 4 auf, an dem zwei Seitenteile 5, ein Hydraulikzylinder 6 mit einer Kolbenstange 10 und ein Rückhalteelement 22 angeordnet sind. Am Trägerelement 4 liegen Gewebeklammern 1 mit ihren Eingriffsabschnitten 3 in einer Vorratsstellung 7 auf, was in Fig. 2 teilweise dargestellt ist.

Die Seitenteile 5 besitzen jeweils eine Ausnehmung 39, deren Hauptteil rechteckig ist und dazu bestimmt ist, die Eingriffsabschnitte 3 der Gewebeklammern 1 in der Vorratsstellung 7 aufzunehmen und oben und unten zu führen. Außerdem wird in der Ausnehmung 39 das jeweilige Rotorelement 9 aufgenommen.

Die Gewebeklammern 1 liegen in Vorratsstellung 7 im Wesentlichen als Stapel aufeinander, wobei die Ebenen 7a, in denen der der Hauptabschnitt 2 und die Eingriffsabschnitte 3 liegen, parallel zueinander sind. Darüber hinaus liegen die Ebenen 7a im Wesentlichen senkrecht auf die Achse 6a des Hydraulikzylinders 6, die auch gleichzeitig die Längsachse der Vorrichtung darstellt. Dies ermöglicht es, dass die Gewebeklammern 1 in Vorratsstellung 7 so angeordnet sein können, dass sich in dem Raum, der zwischen den Eingriffsabschnitten 3 der Gewebeklammern 1 in Vorratsstellung 7 gebildet wird, zumindest teilweise der Hydraulikzylinder 6 bzw. die Kolbenstange 10 befinden können. Dies ermöglicht einen sehr kompakten und platzsparenden Aufbau.

Die Gewebeklammern 1 in Vorratsstellung 7 werden von einem Vorratsschlitten 8 gegen zwei Rotorelemente 9 gedrückt, welche mit einer Welle 12 verbunden sind. Die dafür notwendige Vorspannung wird durch zwei Vorratsfedern 11 erzeugt. Diese sind als abgewickelte Spiralfedern ausgebildet, je ein Ende ist an der Welle 12 befestigt, die anderen Enden am Vorratsschlitten 8.

Jedes der beiden Rotorelemente 9 ist drehbar gelagert und weist eine Auflagefläche 13 mit einer Auflagenase 16 für einen Eingriffsabschnitt 3 einer Gewebeklammer 1 und eine Vertiefung 14 auf. In die Vertiefung 14 kann eine Rotorfeder 15 eingreifen, um ein Einrasten zu ermöglichen, um Positionen der Rotorelemente 9 zu definieren. Die Rotorfeder 15 ist als Fortsatz des Seitenteils 5 ausgebildet.

Ein Schieber 17 ist an der Kolbenstange 10 befestigt und weist zwei Vorschubbereiche 19 mit Nasen 18 auf. Zwischen den Vorschubbereichen 19 ist eine mittige, rechteckige Ausnehmung 20. Über den Hydraulikzylinder 6 ist der Schieber 17 entlang der Längsachse 6a verschiebbar.

In einer ersten Orientierung der Rotorelemente 9 zeigen die Auflageflächen 13 in Richtung der Gewebeklammern 1 in Vorratsstellung 7. Der Vorratsschlitten 8 drückt dabei eine erste Gewebeklammer 1 an die Auflageflächen 13. Nun können die Rotorelemente 9 um etwa 90° gedreht werden. Dabei wird die aufliegende Gewebeklammer 1 durch die Auflagenasen 16 mitgedreht, um eine Arbeitsstellung 29 zu erreichen, in der sie in einer weiteren Ebene 29a angeordnet ist, die parallel zur Längsachse 6a ist. Beim ersten Teil der Drehung wird die erste Gewebeklammer 1 durch den Vorratsschlitten 8 gegen die Auflageflächen 13 gepresst, beim zweiten Teil der Drehung wird die Flächenpressung durch die Rotorfeder 15 aufrechterhalten, um die erste Gewebeklammer 1 in einer definierten Position zu halten. Der Hydraulikzylinder 6 befindet sich zu diesem Zeitpunkt in einer eingefahrenen Position, der Schieber 17 befindet sich daher hinter dem Hauptabschnitt 2 der ersten Gewebeklammer 1.

Nun kann der Hydraulikzylinder 6 ausgefahren werden. Dabei nimmt der Schieber 17 die erste Gewebeklammer 1 mit. Die Position am Schieber 17 wird gesichert, indem eine Auswurffeder 21 die erste Gewebeklammer 1 gegen die Nase 18 drückt. Gleichzeitig werden durch den Vorschub die Auswurffedern 21 immer stärker vorgespannt.

Die erste Gewebeklammer 1 wird nun gegen das Rückhalteelement 22 gedrückt, welches mittig und an die Breite der Ausnehmung 20 des Schiebers 17 angepasst ist. Dadurch wird die erste Gewebeklammer 1 am Hauptabschnitt 2 zu einem Rechteck verformt.

Bei anschließendem Einfahren des Hydraulikzylinders 6 wird die Vorspannung der Auswurffeder 21 auf die erste Gewebeklammer 1 übertragen und somit vom Rückhalteelement 22 abgestreift.

Die Drehung der Rotorelemente 9 erfolgt je über ein an ihnen angelenktes Pleuel 23, das einen ersten Zapfen 30 aufweist, der in eine exzentrische Bohrung 31 des jeweiligen Rotorelements 9 eingreift. Je ein mit dem Schieber 17 verbundener Verbindungsschieber 24 weist ein Langloch 25 auf, in welches ein zweiter Zapfen 26 des Pleuels 23 eingreift. Durch die Verbindung in einem Langloch 25 wird erreicht, dass die Drehung der Rotorelemente 9 jeweils in einer zweiten Phase der Bewegung des Schiebers 17 erfolgt.

In der Folge werden die weiteren Bauteile der erfindungsgemäßen Vorrichtung kurz erläutert:
Abdeckplatten 32 sind seitlich außerhalb der Seitenteile 5 angebracht, um deren Ausnehmungen 33 abzudecken. Die Kolbenstange 10 ist über Dichtungen 34 gegenüber dem Hydraulikzylinder 6 abgedichtet, der über Montageelemente 35 am Trägerelement 4 befestigt ist. Hydraulikanschlüsse 36 und 37 dienen zur Zufuhr des Hydraulikmediums, um die Vorrichtung zu steuern. Mit 38 ist eine Befestigungsklammer bezeichnet.

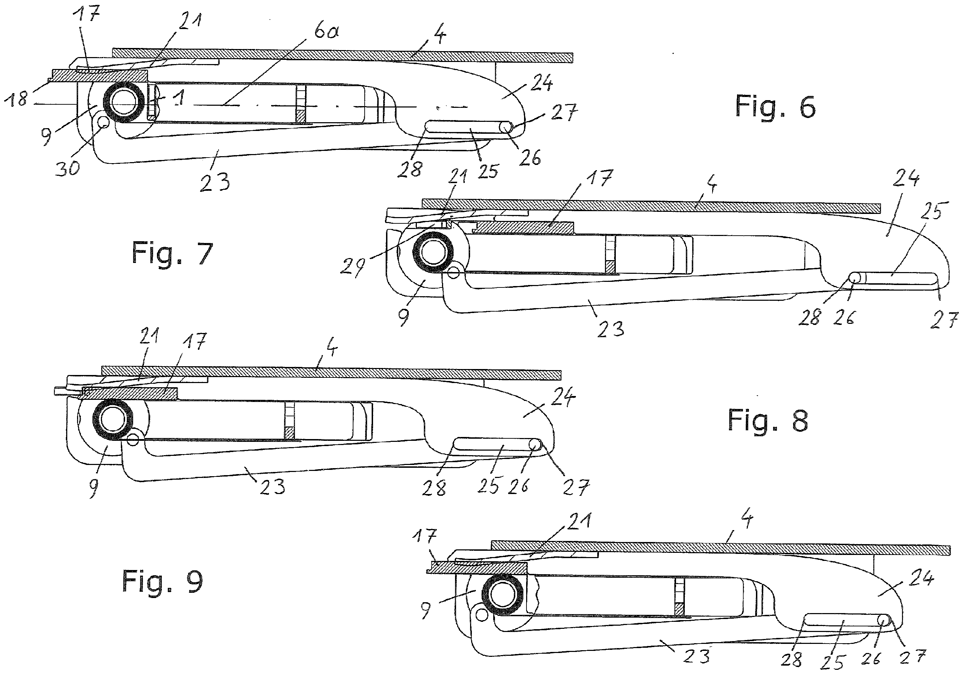

Zur Veranschaulichung des Arbeitsvorgangs der Vorrichtung ist in den Fig. 6 bis Fig. 9 jeweils der gleiche Längsschnitt in unterschiedlichen Positionen des Hydraulikzylinders 6 dargestellt.

Fig. 6 zeigt den Ausgangszustand mit Hydraulikzylinder 6 mit vollständig ausgefahrener Kolbenstange 10. Der Zapfen 26 befindet sich im ausfahrseitigen Anschlag 27 des Langlochs 25, die Auflagefläche 13 der Rotorelemente 9 ist parallel zur Längsachse 6a. Der Schieber 17 befindet sich in der vollständig ausgefahrenen Position, in der gerade eine hier nicht dargestellte Gewebeklammer 1 ausgestoßen worden ist.

Ausgehend von dieser Stellung wird die Kolbenstange 10 eingefahren, der Schieber 17 bewegt sich in der Darstellung nach rechts, der mit ihm verbundene Verbindungsschieber 24 bewegt sich ebenfalls mit. Im ersten Teil des Ausfahrvorgangs wird keine Bewegung auf das Pleuel 23 übertragen, da sich der Zapfen 26 in Richtung des einfahrseitigen Anschlags 28 des Langlochs 25 des Verbindungsschiebers 24 bewegt. Nach etwa dem halben Hub ist der einfahrseitige Anschlag 28 erreicht, und das Pleuel 23 wird nun mit dem Schieber 17 und dem Verbindungsschieber 24 mitbewegt. Dadurch werden die Rotorelemente 9 um etwa einen rechten Winkel gedreht, so dass eine erste Gewebeklammer 1, die in Vorratsstellung auf der Auflageflächen 13 aufliegt aus einer in Fig. 6 senkrechten Stellung in eine waagrechte Stellung gedreht wird, in der sie in der Ebene des Schiebers 17 liegt. Durch die Auflagenasen 16 der Rotorelemente 9 wird die an den Auflageflächen 13 aufliegende Gewebeklammer 1 mitgenommen. Da sich der Schieber 17 zu diesem Zeitpunkt bereits hinter die Rotorelemente 9 zurückgezogen hat, ist die Bewegungsbahn für die Gewebeklammer 1 frei.

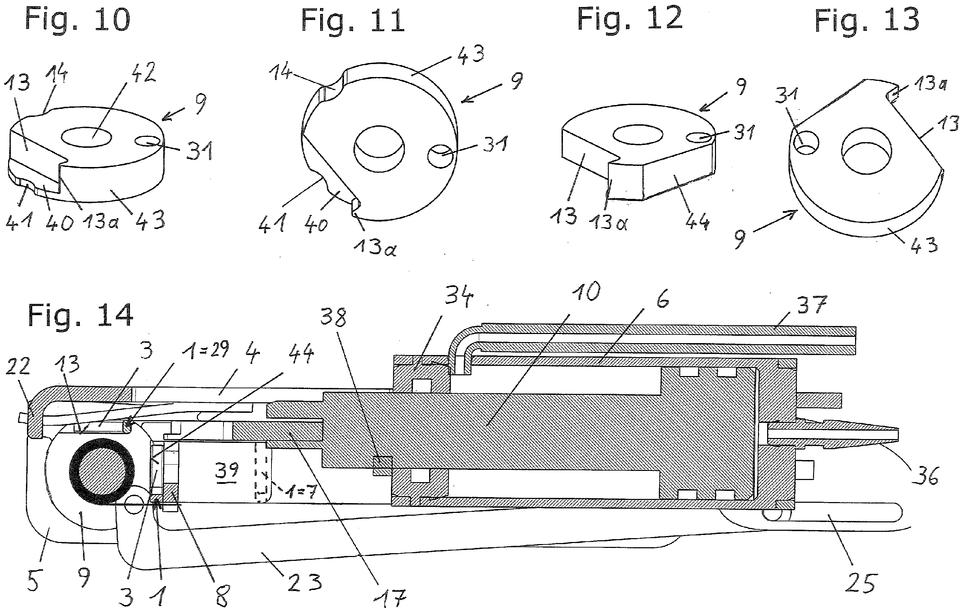

Die so erreichte Stellung mit vollständig eingefahrener Kolbenstange 10 ist in Fig. 7 gezeigt. Nun kann die Kolbenstange 10 wieder aus dem Hydraulikzylinder 6 ausgefahren werden. Es bewegen sich wieder der Schieber 17 mit den Verbindungsschiebern 24 in Richtung der Position der Fig. 6. Dabei wird wieder anfänglich keine Bewegung auf die Pleuel 23 übertragen, da der Zapfen 26 im Langloch 25 in Richtung des ausfahrseitigen Anschlags 27 geschoben wird. Ist der ausfahrseitige Anschlag 27 erreicht, was in der Fig. 8 dargestellt ist, bewegen sich die Pleuel 23 mit der Kolbenstange 10 mit, wodurch die Rotorelemente 9 wieder in die Stellung aus Fig. 6 zurückgedreht werden, die der in der Fig. 10 dargestellten Stellung entspricht. Während der Ausfahrbewegung der Kolbenstange 10, die von Fig. 7 ausgeht und in Fig. 9 endet, wird der Schieber 17 vorgeschoben um letztlich die in der Phase zwischen Fig. 6 und Fig. 7 eingelegte Gewebeklammer 1 zu verformen und auszustoßen. Die Rotorelemente 9 sind in der in der Fig. 9 gezeigten Stellung wieder in der Position, um eine weitere Gewebeklammer 1 aufzunehmen.

Beim Vorschub des Schiebers 17 werden über die Gewebeklammer 1 in Arbeitsstellung 29 die Auswurffedern 21 vorgespannt, wobei die Nasen 18 am Schieber 17 die Gewebeklammer 1 in Arbeitsstellung 29 unten abstützen, so dass die Gewebeklammer 1 beidseitig geführt ist. Beim Zurückziehen des Schiebers 17 entfällt die Abstützung der Gewebeklammer 1 von unten, so dass die Auswurffedern 21 diese nach unten drücken und vom Rückhalteelement 22 abstreifen.

Der Arbeitszyklus kann nach Erreichen der in der Fig. 9 gezeigten Stellung neu beginnen.

In den Fig. 10 bis Fig. 13 sind zwei unterschiedliche Ausführungsvarianten eines Rotorelements 9 dargestellt. Die erste Ausführungsvariante, die in den Fig. 10 und Fig. 11 dargestellt ist, entspricht dabei dem in den Ausführungsvarianten der Fig. 1 bis Fig. 9 verwendeten und dargestellten Rotorelement 9.

Diese erste Ausführungsvariante besitzt eine Auflagefläche 13, die einen Teil eines Kreissegments in Bezug auf den Umfang 43 bildet. An einem Ende ist eine Nase 13a vorgesehen, um die Gewebeklammer 1, die in ihrer Vorratsstellung mit ihrem Eingriffsabschnitt 3 an der Auflagefläche 13 anliegt, bei der Drehung des Rotorelements 9 mitzunehmen. Die Auflagefläche 13 erstreckt sich nicht über die gesamte Dicke des Rotorelements 9. an einer Seite bleibt ein Steg 40 stehen, der den Eingriffsabschnitt 3 der anliegenden Gewebeklammer 1 seitlich führt. Im Bereich dieses Stegs 40 ist eine erste Vertiefung 41 vorgesehen und vom Mittelpunkt des Rotorelementes 9 aus gesehen rechtwinklig dazu ist eine zweite Vertiefung 14 im Umfang 43 ausgebildet. Die beiden Vertiefungen 41, 14 dienen dazu, dass die Rotorfeder 15 einrastet und die Endpositionen der Verdrehung des Rotorelements 9 festlegt. Die exzentrische Bohrung 31 dient der Verdrehung durch das Pleuel 23. Die Bohrung 42 ist zum Aufstecken auf die Welle 12 vorgesehen.

Bei der alternativen Ausführungsvariante, die in den Fig. 12 und Fig. 13 dargestellt ist, erstreckt sich die Auflagefläche 13 über die gesamte Dicke des Umfangs 43. Die seitliche Führung wird dabei von den Abdeckplatten 32 übernommen. Zusätzlich zu der Auflagefläche 13 ist bevorzugt rechtwinkelig dazu eine weitere Anlagefläche 44 am Umfang vorgesehen, an der die Gewebeklammern 1 in der Vorratsstellung 7 anliegen, wenn die Auflagefläche 13 in die Auswurfstellung (parallel zu der Längsachse 6a) gedreht ist. Bei dieser Ausführungsvariante definieren die an der Auflagefläche 13 bzw. der Anlagefläche 44 anliegenden und durch die Vorratsfedern 11 angedrückten Eingriffsabschnitte 3 der Gewebeklammern 1 die Endpositionen der Verdrehung des Rotorelements 9. Vertiefungen sind daher hier nicht erforderlich.

Fig. 14 zeigt einen Längsschnitt einer Ausführungsvariante der erfindungsgemäßen Vorrichtung unter Verwendung der Rotorelemente 9 der Fig. 12 und Fig. 13. Diese Variante entspricht weitgehend der in den Fig. 1 bis Fig. 9 dargestellten Ausführungsvariante. Es ist ersichtlich, dass der Eingriffsabschnitt 3 einer ersten Gewebeklammer 1 auf der Auflagefläche 13 bereits in in der Arbeitsstellung 29 vorliegt. Eine weitere Gewebeklammer 1 liegt mit ihrem Eingriffsabschnitt 3 an der weiteren Anlagefläche 44 an und wird vom Vorratsschieber 8 angedrückt, so dass das Rotorelement 9 in dieser Stellung federnd fixiert ist. Nach dem Auswerfen der ersten Gewebeklammer 1 wird das Rotorelement 9 im Uhrzeigersinn zurückgedreht, wobei jedoch der Vorratsschieber 8 samt den im Vorratsstellung 7 befindlichen Gewebeklammern 1 zunächst gegen den Widerstand der Vorratsfedern 11 zurückgedrückt werden muss.

Aus Fig. 14 ist auch ersichtlich, dass sich die Eingriffsabschnitte 3 der Gewebeklammern 1 im Vorratsstellung 7 (eine Gewebeklammer 1 ist mit unterbrochenen Linien entsprechend dargestellt) deutlich über den unteren Bereich der Kolbenstange 10 hinaus nach oben erstrecken, was bedeutet, dass sich die Kolbenstange 10 in der ausgefahrenen Stellung innerhalb des rechteckigen Raums bewegt, der an drei Seiten von dem Hauptabschnitt 2 und den beiden Eingriffsabschnitten 3 der Gewebeklammer 1 aufgespannt ist.

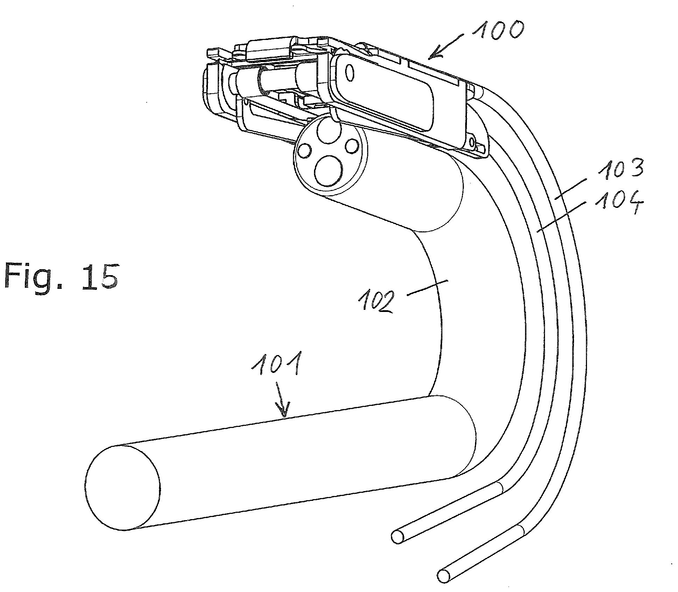

In Fig. 15 ist eine erste Variante des Einsatzes einer erfindungsgemäßen Vorrichtung dargestellt. Dabei ist der Kopfteil 100 abnehmbar auf einem üblichen Endoskop 101 mit einer Abwinkelung 102 aufgesetzt und wird über Hydraulikschläuche 103 und 104 gesteuert, die parallel zum Endoskop in eine Körperöffnung eingeführt werden.

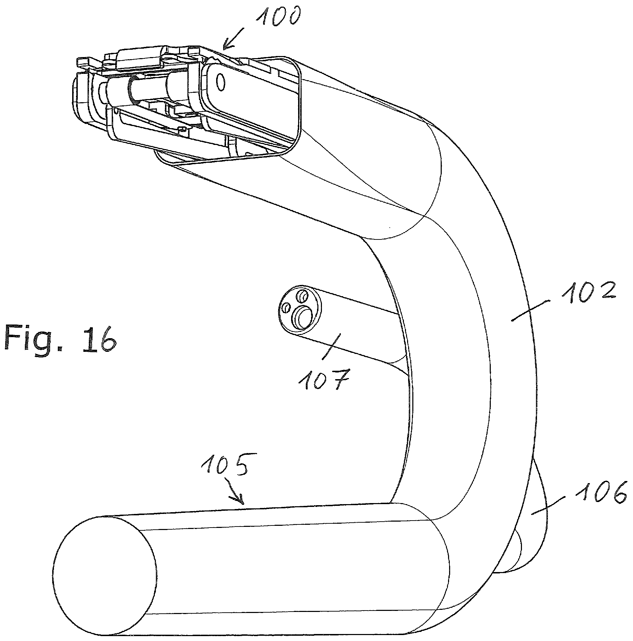

Fig. 16 zeigt eine alternative Variante des Einsatzes der erfindungsgemäßen Vorrichtung, bei der der Kopfteil 100 die Spitze eines eigenständigen flexiblen Instruments 105 bildet. Dieses besitzt gemäß üblicher Bauart mindestens einen Kanal, durch den ein Endoskop 107 mit einer Abwinkelung 102 einschiebbar ist. Das Endoskop 107 ist mit einer eigenständigen Abwinkelung 106 seitlich herausgeführt, um den Vorgang des Verklammerns beobachten zu können.

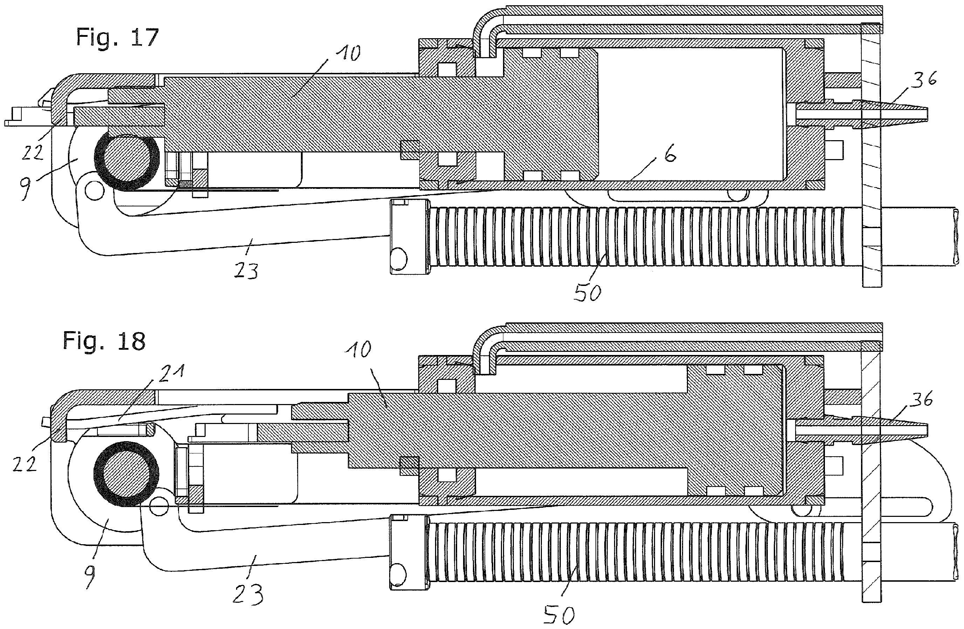

Die Ausführungsvariante der Fig. 17 und Fig. 18 unterscheidet sich von den oben beschriebenen Varianten dadurch, dass zwei Kanäle 50, 51 unterhalb des Hydraulikzylinders 6 vorgesehen sind. Die Kanäle 50, 51 sind zum distalen Ende hin nach außen gerichtet, also divergent, wobei der Winkel der Divergenz vorzugsweise einstellbar ist. Wenn nun durch die Kanäle 50 und 51 jeweils Halteinstrumente vorgeschoben werden, dann treten diese leicht nach außen gerichtet aus, wobei sich somit variable Ausmaß durch den Abstand zur Spitze, also den Raum unterhalb der Gewebeklammern 1 in ihrer Vorratsstellung 7 noch vergrößert.

Durch die besondere Gestaltung der Vorrichtung ist es möglich die Kanäle 50 und 51 innerhalb des kreisförmigen Querschnitts anzuordnen, der durch die übrigen Bauteile definiert ist.

Die vorliegende Erfindung ermöglicht es, Gewebe im Körperinneren mit Gewebeklammern zu verbinden, wobei es besonders vorteilhaft ist, dass nacheinander eine Mehrzahl von Gewebeklammern angewendet werden können, ohne die Notwendigkeit, die Vorrichtung dazu aus dem Körper des Patienten zu ziehen und danach erneut einzuführen.

## Patentansprüche

1. Vorrichtung zum Verbinden von Körpergeweben mit einem in eine Körperöffnung einschiebbaren Kopfteil (100) mit einer Längsachse (6a), wobei in dem Kopfteil (100) eine Mehrzahl von Gewebeklammern (1) in einer Vorratsstellung (7) aufgenommen sind, die aus einem linearen Hauptabschnitt (2) und zwei davon senkrecht abstehenden Eingriffsabschnitten (3) bestehen und die jeweils parallel zu einer ersten Ebene (7a) ausgerichtet sind, die im Wesentlichen senkrecht zur Längsachse (6a) ist, wobei ein Schieber (17) vorgesehen ist, um die in einer Arbeitsstellung (29) vorliegende Gewebeklammer (1) in einer weiteren Ebene (29a), die im Wesentlichen senkrecht zur ersten Ebene (7a) orientiert ist, vorzuschieben und im Zusammenwirken mit einem Rückhalteelement (22) in eine Klammerstellung zu verformen, **dadurch gekennzeichnet, dass** zwei Rotorelemente (9) vorgesehen sind, die um eine zu den Hauptabschnitten (2) der Gewebeklammern (2) parallele Achse schwenkbar sind und die jeweils eine Auflagefläche (13) für einen Eingriffsabschnitt (3) einer Gewebeklammer (1) aufweisen, um diese aus der Vorratsstellung (7) in die Arbeitsstellung (29) zu drehen, in der die Gewebeklammer (1) in der weiteren Ebene (29a) angeordnet ist.

2. Vorrichtung nach Anspruch 1, **dadurch gekennzeichnet, dass** jeweils eine Rotorfeder (15) an einem Rotorelement (9) angreift, um dessen Bewegung in zwei Rotationsstellungen einrastbar auszuführen, und dass vorzugsweise die Rotorelemente (9) mit einer Welle (12) miteinander verbunden sind.

3. Vorrichtung nach Anspruch 2, **dadurch gekennzeichnet, dass** die Gewebeklammern (1) in Vorratsstellung (7) durch zumindest eine Vorratsfeder (11) über einen Vorratsschlitten (8) gegen das Rotorelement (9) vorgespannt wird, wobei die Vorratsfeder (11) vorzugsweise an der Welle (12) der Rotorelemente (9) befestigt ist.

4. Vorrichtung nach einem der Ansprüche 1 bis 3, **dadurch gekennzeichnet, dass** die Rotorelemente (9) durch zumindest ein Pleuel (23) angetrieben werden, das an ihnen angelenkt ist, und welches vorzugsweise über einen Verbindungsschieber (24) mit dem Schieber (17) in Verbindung steht.

5. Vorrichtung nach einem der Ansprüche 1 bis 4, **dadurch gekennzeichnet, dass** die Rotorelemente (9) durch je ein Pleuel (23) angetrieben werden, und vorzugsweise je über einen Verbindungsschieber (24) mit dem Schieber (17) in Verbindung stehen.

6. Vorrichtung nach Anspruch 4 oder 5, **dadurch gekennzeichnet, dass** der bzw. die Verbindungsschieber (24) fest mit dem Schieber (17) verbunden ist/sind, und dass an jedem Verbindungsschieber (24) ein Langloch (25) angeordnet ist, in das je ein Zapfen (26) des Pleuels (23) eingreift.

7. Vorrichtung nach einem der Ansprüche 1 bis 6, **dadurch gekennzeichnet, dass** die Gewebeklammern (1) in Vorratsstellung (7) in Ausnehmungen (39) von Seitenteilen (5) geführt sind.

8. Vorrichtung nach einem der Ansprüche 1 bis 6, **dadurch gekennzeichnet, dass** das Rotorelement (9) eine Anlagefläche (44) aufweist, die vorzugsweise rechtwinkelig zur Auflagefläche (13) ist.

9. Vorrichtung nach einem der Ansprüche 1 bis 8, **dadurch gekennzeichnet, dass** ein Schieber (17) auf der, den Eingriffsabschnitten (3) abgewandten, Seite der Hauptabschnitte (2) der Gewebeklammern (1) in Richtung der Längsachse (6a) beweglich angeordnet ist, der an seiner vorderen Stirnseite zwei seitliche Vorschubbereiche (19) aufweist, zwischen denen eine Ausnehmung (20) vorgesehen ist, und dass das Rückhalteelement (22) in die Ausnehmung (20) des Schiebers (17) eingreift, um beim Vorschub des Schiebers (17) den linearen Hauptabschnitt (2) einer dazwischenliegenden Gewebeklammer (1) zu verformen.

10. Vorrichtung nach Anspruch 9, **dadurch gekennzeichnet, dass** eine Auswurffeder (21) so angeordnet ist, dass diese eine verformte Gewebeklammer (1) vom Rückhalteelement (22) abstreift und dass die Auswurffeder (21) im kräftefreiem Zustand vorzugsweise in den Bewegungsbereich des Schiebers (17) ragt.

11. Vorrichtung nach Anspruch 10, **dadurch gekennzeichnet, dass** die Auswurffeder (21) während dem Vorschub des Schiebers (17) vorgespannt wird, und während einem Rückschub des Schiebers (17) die Vorspannung auf die Gewebeklammer (1) überträgt.

12. Vorrichtung nach einem der Ansprüche 9 bis 11, **dadurch gekennzeichnet, dass** der Schieber (17) plattenförmig ausgebildet ist und der Bewegungsbereich des Schiebers (17) in einer weiteren Ebene (29a) liegt, die im Bereich der Spitzen der Eingriffsabschnitte (3) der Gewebeklammern (1) in Vorratsstellung (7) liegt, und besonders vorzugsweise im Wesentlichen parallel und mit konstantem Abstand zu den Hauptabschnitten (2) der Gewebeklammern (1) in Vorratsstellung (7) liegt.

13. Vorrichtung nach einem der Ansprüche 1 bis 12, **dadurch gekennzeichnet, dass** ein Hydraulikzylinder (6) und/oder eine Kolbenstange (10) zumindest teilweise im Inneren des Raums angeordnet sind, der zwischen den Eingriffsabschnitten (3) der Gewebeklammern (1) in der Vorratsstellung (7) liegt, und vorzugsweise, dass die Kolbenstange (10) mit dem Schieber (17) fest verbunden ist.

14. Vorrichtung nach einem der Ansprüche 1 bis 13, **dadurch gekennzeichnet, dass** der Kopfteil (100) als Aufsatz auf ein Endoskop (101) ausgebildet ist oder einstückig mit einem flexiblen Instrument (105) verbunden ist, das vorzugsweise einen Kanal für ein Endoskop (107) aufweist.

15. Vorrichtung nach einem der Ansprüche 1 bis 14, **dadurch gekennzeichnet, dass** mindestens ein Kanal (50, 51) zur Aufnahme eines Halteinstruments vorgesehen ist und dass vorzugsweise zwei Kanäle (50, 51) vorgesehen sind, die vorzugsweise nach vorne geringfügig auseinanderlaufen, wobei besonders vorzugsweise der Winkel zwischen den Kanälen (50, 51) steuerbar ist.

## Claims

1. Device for connecting body tissues, having a head part (100), which can be pushed into a body opening and has a longitudinal axis (6a), wherein a plurality of tissue staples (1) are accommodated in the head part (100) in a storage position (7), which tissue staples (1) consist of a linear main section (2) and two engagement sections (3) projecting perpendicularly therefrom and are each aligned in parallel to a first plane (7a) which is substantially perpendicular to the longitudinal axis (6a), wherein a slider (17) is provided in order to advance the tissue staple (1), which is present in the working position (29), into a further plane (29a), which is aligned substantially perpendicularly to the first plane (7a), and to deform it into a clamping position in interaction with a retaining element (22), **characterised in that** two rotor elements (9) are provided, which are pivotable about an axis parallel to the main sections (2) of the tissue staples (1) and which each have a bearing surface (13) for an engagement section (3) of a tissue staple (1) in order to rotate said tissue staple (1) from the storage position (7) into the working position (29) in which the tissue staple (1) is arranged in the further plane (29a).

2. Device according to claim 1, **characterised in that** a rotor spring (15) engages in each case on a rotor element (9) in order to carry out its movement in two rotational positions so as to be latchable, and **in that** the rotor elements (9) are preferably connected to one another by means of a shaft (12).

3. Device according to claim 2, **characterised in that** the tissue staples (1) in the storage position (7) are pretensioned against the rotor element (9) by at least one storage spring (11) via a storage slide (8), wherein the storage spring (11) is preferably fastened to the shaft (12) of the rotor elements (9).

4. Device according to one of claims 1 to 3, **characterised in that** the rotor elements (9) are driven by at least one connecting rod (23) which is articulated to them and which is preferably connected to the slider (17) via a connecting slider (24).

5. Device according to one of claims 1 to 4, **characterised in that** the rotor elements (9) are each driven by a connecting rod (23) and are preferably each connected to the slider (17) via a connecting slider (24).

6. Device according to claim 4 or 5, **characterised in that** the connecting slider or connecting sliders (24) is or are firmly connected to the slider (17), and **in that** an elongated hole (25) is arranged on each connecting slider (24), in each of which a pin (26) of the connecting rod (23) engages.

7. Device according to one of claims 1 to 6, **characterised in that** the tissue staples (1) are guided, in the storage position (7), in recesses (39) of side parts (5).

8. Device according to one of claims 1 to 6, **characterised in that** the rotor element (9) has a contact surface (44) which is preferably at a right angle to the bearing surface (13).

9. Device according to one of claims 1 to 8, **characterised in that** a slider (17) is arranged on the side of the main sections (2) of the tissue staples (1) facing away from the engagement sections (3) so as to be movable in the direction of the longitudinal axis (6a), said slider (17) having two lateral feed regions (19) on its front end face, between which a recess (20) is provided, and **in that** the retaining element (22) engages in the recess (20) of the slider (17) in order to deform the linear main section (2) of an interposed tissue staple (1) when the slider (17) is advanced.

10. Device according to claim 9, **characterised in that** an ejection spring (21) is arranged in such a way that it strips off a deformed tissue staple (1) from the retaining element (22), and **in that** the ejection spring (21) preferably projects in the force-free state into the range of movement of the slider (17).

11. Device according to claim 10, **characterised in that** the ejection spring (21) is pretensioned during the advance of the slider (17) and transfers the pretension to the tissue staple (1) during a return stroke of the slider (17).

12. Device according to one of the claims 9 to 11, **characterised in that** the slider (17) is formed in a plate-shaped manner and the range of movement of the slider (17) lies in a further plane (29a) which lies in the storage position (7) in the region of the tips of the engagement sections (3) of the tissue staples (1), and lies particularly preferably substantially parallel and at a constant distance from the main sections (2) of the tissue staples (1) in the storage position (7).

13. Device according to one of claims 1 to 12, **characterised in that** a hydraulic cylinder (6) and/or a piston rod (10) are at least partially arranged inside the space disposed between the engagement sections (3) of the tissue staples (1) in the storage position (7), and preferably **in that** the piston rod (10) is fixedly connected to the slider (17).

14. Device according to one of claims 1 to 13, **characterised in that** the head part (100) is designed as an attachment to an endoscope (101) or is integrally connected to a flexible instrument (105) which preferably has a channel for an endoscope (107).

15. Device according to one of claims 1 to 14, **characterised in that** at least one channel (50, 51) is provided for receiving a holding instrument, and **in that** preferably two channels (50, 51) are provided which preferably diverge slightly towards the front, wherein the angle between the channels (50, 51) is particularly preferably controllable.

## Revendications

1. Dispositif pour relier des tissus corporels avec une tête (100) qui se glisse dans l'ouverture anatomique, ayant un axe longitudinal (6a),
- la tête (100) recevant un ensemble d'agrafes (1) en position de réserve (7), ces agrafes se composant d'un segment principal (2), linéaire et de deux segments de pénétration (3) en saillie perpendiculairement à celui-ci et qui sont orientés chacun parallèlement à un premier plan (7a) pratiquement perpendiculaire à l'axe longitudinal (6a),
- un coulisseau (17) étant prévu pour pousser l'agrafe (1) qui est en position active (29) dans un autre plan (29a) pratiquement perpendiculaire au premier plan (7a), et en coopérant avec un élément de retenue (22), pour la déformer en position d'agrafe,
dispositif **caractérisé en ce qu'**il comporte
- deux éléments de rotor (9) pivotants autour d'un axe parallèle aux segments (2) principaux des agrafes (1), et ayant une surface de réception (13) pour un segment de pénétration (3) d'une agrafe (1), pour tourner celle-ci de sa position de réserve (7) dans sa position active (29) dans laquelle l'agrafe (1) se trouve dans l'autre plan (29a).

2. Dispositif selon la revendication 1,
**caractérisé en ce qu'**
un ressort de rotor (15) respectif agit sur un élément de rotor (9) pour effectuer son mouvement d'accrochage dans deux positions de rotation, et de préférence les éléments de rotor (9) sont reliés l'un à l'autre par un axe (12).

3. Dispositif selon la revendication 2,
**caractérisé en ce que**
les agrafes (1) en position de réserve (7) sont précontraints par au moins un ressort de réserve (11) par un chariot de réserve (8) contre l'élément de rotor (9),
le ressort de réserve (11) étant fixé de préférence à l'axe (12) des éléments de rotor (9).

4. Dispositif selon l'une des revendications 1 à 3,
**caractérisé en ce que**
les éléments de rotor (9) sont entraînés par au moins une bielle (23) articulée sur ceux-ci et reliée, de préférence au coulisseau (17) par un coulisseau de liaison (24).

5. Dispositif selon l'une des revendications 1 à 4,
**caractérisé en ce que**
les éléments de rotor (9) sont entraînés chacun par une bielle (23) et de préférence ils sont reliés au coulisseau (17) par un coulisseau de liaison (24) respectif.

6. Dispositif selon l'une des revendications 4 ou 5,
**caractérisé en ce que**
le ou les coulisseaux de liaison (24) sont reliés solidairement au coulisseau (17), et
chaque coulisseau de liaison (24) a un trou oblong (25) dans lequel pénètre un téton (26) de la bielle respective (23).

7. Dispositif selon l'une des revendications 1 à 6,
**caractérisé en ce que**
en position de réserve (7) les agrafes (1) sont guidées dans les évidements (39) de pièces latérales (5).

8. Dispositif selon l'une des revendications 1 à 6,
**caractérisé en ce que**
l'élément de rotor (9) a une surface d'appui (44) qui est, de préférence, perpendiculaire à la surface réceptrice (13).

9. Dispositif selon l'une des revendications 1 à 8,
**caractérisé en ce que**
le coulisseau (17) est mobile dans la direction de l'axe longitudinal (6a) sur le côté des segments principaux (2) des agrafes (1) à l'opposé des segments de pénétration (3), le coulisseau comportant sur son côté frontal avant, deux zones latérales de poussée (19) ayant entre lesquelles un évidement (20), et
l'élément de retenue (22) pénètre dans l'évidement (20) du coulisseau (17) pour, lors de l'avancée du coulisseau (17), déformer le segment principal linéaire (2) en une agrafe (1) venant en position intermédiaire.

10. Dispositif selon la revendication 9,
**caractérisé en ce qu'**il comporte
un ressort éjecteur (21) qui râcle l'agrafe déformée (1) de l'élément de retenue (22), et
le ressort éjecteur (21) à l'état hors contrainte, pénètre, de préférence, dans la zone de mouvement du coulisseau (17).

11. Dispositif selon la revendication 10,
**caractérisé en ce que**
le ressort éjecteur (21) est précontraint pendant le mouvement d'avancée du coulisseau (17) et il transmet sa précontrainte à l'agrafe (1) pendant la course de retour du coulisseau (17).

12. Dispositif selon l'une des revendications 9 à 11,
**caractérisé en ce que**
le coulisseau (17) est en forme de plaque et la plage de mouvement du coulisseau (17) est dans un autre plan (29a) qui se situe dans la zone des pointes des segments de pénétration (3) des agrafes (1) en position de réserve (7) et d'une manière particulièrement préférentielle, il est pratiquement parallèle et à distance constante des segments principaux (2) des agrafes (1) en position de réserve (7).

13. Dispositif selon l'une des revendications 1 à 12,
**caractérisé en ce qu'**
un vérin hydraulique (6) et/ou une tige de piston (10) sont au moins en partie à l'intérieur du volume situé entre les segments de pénétration (3) des agrafes (1) en position de réserve (7) et de préférence la tige de piston (10) est reliée solidairement au coulisseau (17).

14. Dispositif selon l'une des revendications 1 à 13,
**caractérisé en ce que**
la tête (100) est réalisée comme prolongement d'un endoscope (101) ou est reliée en une seule pièce à un instrument souple (105) qui comporte, de préférence, un canal pour un endoscope (107).

15. Dispositif selon l'une des revendications 1 à 14,
**caractérisé en ce qu'**il comporte
au moins un canal (50, 51) pour recevoir un instrument de maintien et de préférence deux canaux (50, 51) qui s'écartent de préférence légèrement vers l'avant et d'une manière particulièrement préférentielle, l'angle entre les canaux (50, 51) se commande.
